# EUROPEAN PATENT SPECIFICATION

(11) **EP 2 037 917 B1**
(45) Date of publication and mention of the grant of the patent: **08.10.2014**
(21) Application number: 07851323.1
(22) Date of filing: 07.12.2007
(51) Int. Cl.: A61K 31/44, A61K 45/06, A61K 31/4178, A61K 31/4422, A61P 9/00, A61P 9/10, A61P 9/04

(54) **PHARMACEUTICAL COMPOSITION COMPRISING AMLODIPINE AND LOSARTAN**
PHARMAZEUTISCHE ZUSAMMENSETZUNG MIT AMLODIPIN UND LOSARTAN
COMPOSITION PHARMACEUTIQUE COMPRENANT DE L'AMLODIPINE ET DU LOSARTAN

(30) Priority: 08.12.2006 KR 20060124552
(43) Date of publication of application: 25.03.2009
(73) Proprietor: Hanmi Science Co., Ltd., Gyeonggi-do 445-813 (KR)
(72) Inventor: WOO, Jong Soo, Jangan-gu, Suwon-si, Gyeonggi-do 440-710 (KR); YI, Hong Gi, Jangan-gu, Suwon-si Gyeonggi-do 440-728 (KR); CHI, Moon Hyuk, Suwon-si, Gyeonggi-do 440-829 (KR); KIM, Kyeong Soo, Suwon-si, Gyeongg-do 441-340 (KR)
(74) Representative: Grünecker, Kinkeldey, Stockmair & Schwanhäusser
(86) International application number: PCT/KR2007/006352
(87) International publication number: WO 2008/069612

(56) References cited:
- EP-A1- 1 314 425
- WO-A1-00/27397
- WO-A1-97/49392
- WO-A1-2008/044862
- WO-A2-2004/075892
- WO-A2-2005/070463
- US-A1- 2006 110 450
- US-A1- 2006 252 805
- US-A1- 2006 252 806

## Description

### Field of the Invention

The present invention relates to a composition for preventing or treating cardiovascular disorders comprising amlodipine or a pharmaceutically acceptable salt thereof and losartan or a pharmaceutically acceptable salt thereof, and a method for preparing the same.

### Background of the Invention

Hypertension can be classified into essential or secondary hypertension, and most (approximately 90 - 95%) of the hypertension belongs to the essential hypertension class. While secondary hypertension is generally treatable by correcting the known causes, essential hypertension, the exact cause of which is not yet elucidated, is generally treated by relaxation therapy, dietary therapy and exercise therapy which are optionally combined with medication. Notable antihypertensive drugs include diuretics, sympatholytic agents and vasodilators. Vasodilators are most widely prescribed antihypertensive drugs, and they are divided into several groups according to their pharmacological action which include ACE (angiotensin converting enzyme) inhibitors, angiotensin II receptor antagonists and calcium channel blockers.

In the treatment of hypertension, it is more important to maintain the blood pressure within a normal range on a consistent basis than to simply lower the blood pressure level itself, for reducing the risks of complications such as coronary heart diseases and cardiovascular diseases, e.g., stroke, heart failure and myocardial infarction. Accordingly, antihypertensive agents should be effective for long-term treatment of hypertension. Further, advanced therapy using a combination of two or more drugs having different pharmacological actions makes it possible to improve preventive or therapeutic effects, while lowering side effects arising from the long term administration of a single drug.

WO-A-2008/044862 relates to a functional combination preparation comprising a dihydropyridine-based calcium channel blocker such as amlodipine and an ARB (angiotensin-2 receptor blocker) such as losartan. In particular, the document relates to chronotherapeutically designed combination formulations for the preparation and treatment of cardiovascular diseases, which are formulated based on xenobiotics and chronotherapy for enabling the two drugs to be chronotherapeutically released, thereby improving the therapeutic activity as compared to the co-administration of each drug in the form of a single tablet, while reducing side effects and maintaining the therapeutic activity as high as possible during the period of time of a day when the risk of a compliance of cardiovascular disease is highest.

WO-A-2005/070463 relates to a pharmaceutical composition comprising enantiomerically pure (S)-amlodipine malate, an ARB and optional other active agents. Moreover, the document is directed to methods for treating, preventing and managing cardiovascular diseases and disorders and symptoms thereof, using the compulsion.

EP-A-1314425 is directed to medicinal compositions for preventing or treating heart failure and discloses compositions containing a HMG-CoA reductase inhibitor selected from the group consisting of pravastatin, simvastatin, lovastatin, pitavsastin and ZD-4522, an angiotensin II receptor antagonist and optionally a calcium, channel blocker.

The Indian patent application 755-MUM-2003 is concerned with a process of making a stable pharmaceutical formulation of tablet comprising an effective amount of (S)-amlodipine besilate salt with pharmaceutically acceptable and compatible (physical and chemical) excipients.

It is the object of the present invention to provide a composition for use in the prevention or treatment of cardiovascular disorders comprising amlodipine or a pharmaceutically acceptable salt thereof and losartan or a pharmaceutically acceptable salt thereof that shows an enhanced stability and dissolution rate.

Said object is solved by:
1. A composition for use in preventing or treating cardiovascular disorders comprising amlodipine or a pharmaceutically acceptable salt thereof, losartan or a pharmaceutically acceptable salt thereof, and pharmaceutically acceptable excipients, wherein amlodipine or a pharmaceutically acceptable salt thereof and losartan or a pharmaceutically acceptable salt thereof are physically separated from each other, said composition being obtainable by a method comprising the steps of granulating amlodipine or a pharmaceutically acceptable salt thereof to obtain an amlodipine granule part, and mixing the amlodipine granule part with a mixture part comprising losartan or a pharmaceutically acceptable salt thereof.
2. The composition according to item 1 for use in preventing or treating cardiovascular disorders according to item 1, which further comprises a coating layer formed on the amlodipine granule part.
3. The composition according to item 1 for use in preventing or treating cardiovascular disorders according to item 1, wherein the amlodipine granule part and the mixture part further comprise pharmaceutically acceptable excipients.
4. The composition according to item 3 for use in preventing or treating cardiovascular disorders according to item 1, wherein the amlodipine granule part comprises amlodipine or a pharmaceutically acceptable salt thereof and pharmaceutically acceptable excipients in amounts corresponding to a weight ratio in the range of 1:10 to 1:60.
5. The composition according to item 1 for use in preventing or treating cardiovascular disorders according to item 1, wherein the pharmaceutically acceptable salt of amlodipine is amlodipine camsylate.
6. The composition according to item 1 for use in preventing or treating cardiovascular disorders according to item 1, wherein the pharmaceutically acceptable salt of losartan is losartan potassium.
7. The composition according to item 1 for use in preventing or treating cardiovascular disorders according to item 1, wherein the cardiovascular disorders are selected from the group consisting of angina pectoris, hypertension, artery vasospasm, deep vein thrombosis, cardiac hypertrophy, cerebral infarct, congestive heart failure and myocardial infarction.
8. A method for preparing the composition of item 1, which comprises the steps of:
   a) wet-granulating and drying a mixture of amlodipine or a pharmaceutically acceptable salt thereof and pharmaceutically acceptable excipients to obtain an amlodipine granule part; and
   b) mixing the amlodipine granule part obtained in step a) with a mixture part comprising losartan or a pharmaceutically acceptable salt thereof and pharmaceutically acceptable excipients.

### Brief Description of the Drawings

The above and other objects and features of the present invention will become apparent from the following description of the invention, when taken in conjunction with the accompanying drawings which respectively show:
Fig. 1: a graph showing the amlodipine dissolution rate, obtained in Test Example 1;
Fig. 2: a graph showing the losartan dissolution rate, obtained in Test Example 2;
Fig. 3: a graph showing the amlodipine dissolution rate, obtained in Test Example 3;
Fig. 4: a graph showing the amlodipine dissolution rate, obtained in Test Example 4; and
Fig. 5: a graph showing the amlodipine dissolution rate, obtained in Test Example 5.

### Detailed Description of the Invention

Amlodipine is the generic name for 3-ethyl-5-methyl-2-(2-aminoethoxy-methyl)-4-(2-chlorophenyl)-6-methyl-1,4-dihydro-3,5-pyridine dicarboxylate, and EP Patent Publication No. 89167 discloses various forms of pharmaceutically acceptable salts of amlodipine. The pharmaceutically acceptable salts of amlodipine used in the present invention may be formed using acids which form non-toxic, pharmaceutically acceptable acid addition salts, which include but are not limited to hydrochloride, hydrobromide, sulphate, phosphate, acetate, maleate, fumarate, lactate, tartrate, citrate, gluconate, besylate and camsylate salts. Among the acid addition salts, amlodipine besylate is currently marketed as Novasc (trade mark), and Korean Patent No. 452491 has disclosed the camsylate salt of amlodipine having improved physical properties, i.e., higher solubility and stability as compared with amlodipine besylate. Accordingly, the most preferred pharmaceutically acceptable salt of amlodipine that can be used in the present invention is amlodipine camsylate. Amlodipine is a long-acting calcium channel blocker which is useful in treating cardiovascular disorders such as agina, hypertension and congestive heart failure. However, it has been reported that prolonged anti-hypertensive therapy with amlodipine often causes side effects such as dose-limiting peripheral edema, especially ankle edema. The amlodipine-induced ankle edema, for example, is believed to be due the the preferential dilation of the precapillary arterioles in the leg and the resultant efflux of fluid into the interstitial space. A daily proposed dose of amlodipine or a pharmaceutically acceptable salt thereof is 0.5~20 mg, preferably 1~10 mg, more preferably 5~10 mg.

Losartan is the generic name for 2-butyl-4-chloro-1-[[2'-(1*H*-tetrazol-5-yl)[1,1'-biphenyl]-4-yl]methyl]-1*H*-imidazol-5-methanol, which has been disclosed in U.S. Patent Nos. 5,608,075; 5,138,069; and 5,153,197. Losartan potassium is currently available as Cozaar (trade mark). Accordingly, the preferred pharmaceutically acceptable salt of losartan that can be used in the present invention is losartan potassium. Losartan blocks the interaction of angiotensin II and its receptor, and is mainly used for treating hypertension, heart failure, ischemic peripheral circulatory disorder, myocardial ischemia (angina pectoris), diabetic neuropathy and glaucoma, and also for preventing the progression of post-myocardial infarction heart failure. Although losartan is reported to cause low incidence of cough or edema, it sometimes induces side effects such as dizziness or orthostatic hypotension. A daily proposed dose of losartan or a pharmaceutically acceptable salt thereof is 0.1~500 mg, preferably 1~200 mg, more preferably 25~200 mg.

The inventive composition comprising amlodipine or a pharmaceutically acceptable salt thereof and losartan or a pharmaceutically acceptable salt thereof can achieve improved preventive or therapeutic effects for cardiovascular disorders, such as angina pectoris, hypertension, artery vasospasm, deep vein, cardiac hypertrophy, cerebral infarct, congestive heart failure and myocardial infarction, as compared with conventional single formulations, while minimizing adverse effects of the two drugs.

However, the combined formulation of amlodipine and losartan of the present invention cannot be prepared simply by mixing the two drugs for the following problems.

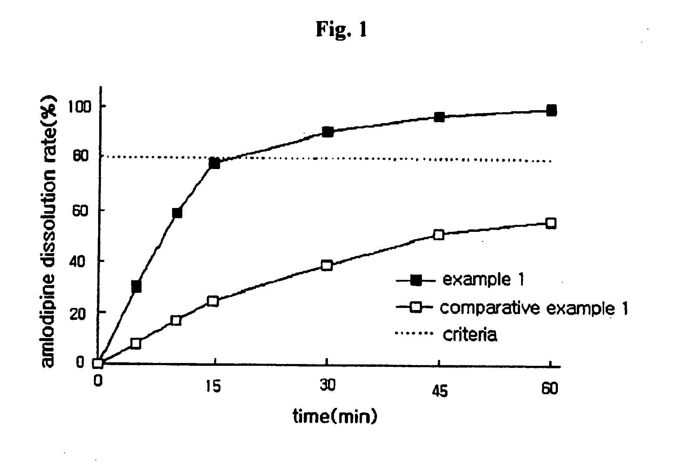

The first problem is gelation of losartan. Losartan dissolves readily in water of at high pH, e.g., pH 4.0 or pH 6.8, but it is very slowly released in an aqueous medicament at low pH (e.g., pH 2.0 or pH 1.2) because of its gelation. Accordingly, the dissolution rate and bioavailability of the combined formulation comprising losartan are expected to be unsatisfactory because the formulation is first exposed to the acidic gastronic fluid having low pH value when orally administered. Further, amlodipine may be locked in the inside of the formulation due to the gelation of losartan. For example, as can be seen in Fig 1, which is the results of Test Example 1, a tablet prepared by simply mixing the two drugs fails to meet the dissolution criteria of amlodipine, i.e., 80% at 30 mins. Therefore, an acceptable combined formulation must be free from the problem of losartan gelation even under a low pH condition.

The second problem is that the drug stability of a simple combined formulation mixing the two drugs decreases rapidly due to the adverse influence of losartan on the drug stability of amlodipine.

Accordingly, the present invention also includes within its scope a combined formulation of amlodipine and losartan in which the contact between the two drugs is minimized by physically separating amlodipine or a pharmaceutically acceptable salt thereof from losartan or a pharmaceutically acceptable salt thereof, thereby improving the dissolution rates and stabilities of amlodipine and losartan.

In accordance with one embodiment of the present invention, the amlodipine-losartan combined formulation may be prepared by using a separate granule or form of amlodipine in order to physically separate amlodipine from losartan in the formulation. That is, the inventive combined formulation may be prepared by a method comprising the steps of 1) granulating amlodipine or a pharmaceutically acceptable salt thereof to obtain separated granules; and 2) mixing the separated granule part with a mixture comprising losartan or a pharmaceutically acceptable salt thereof. The combined formulations of Examples 1 to 4 prepared by this method exhibit an enhanced dissolution rate of amlodipine while maintaining a satisfactory dissolution rate of losartan (*see* Figs. 1 to 3), and also exhibit high drug stability of amlodipine (*see* Table 1), as compared with the combined formulation of Comparative Example 1, which is a tablet obtained using a simple mixture of amlodipine or a pharmaceutically acceptable salt thereof and losartan or a pharmaceutically acceptable salt thereof.

Accordingly, in a further embodiment, the present invention also includes within its scope a composition for preventing or treating cardiovascular disorders comprising amlodipine or a pharmaceutically acceptable salt thereof and losartan or a pharmaceutically acceptable salt thereof, the part comprising amlodipine or a pharmaceutically acceptable salt thereof being separated from the part containing losartan or a pharmaceutically acceptable salt thereof.

In yet another embodiment, the present invention also includes within its scope a composition for preventing or treating cardiovascular disorders comprising amlodipine or a pharmaceutically acceptable salt thereof and losartan or a pharmaceutically acceptable salt thereof, the composition being prepared by separately granulating amlodipine or a pharmaceutically acceptable salt thereof to obtain an amlodipine granule part, and mixing the amlodipine granule part with a mixture comprising losartan or a pharmaceutically acceptable salt thereof. The composition of the present invention may further comprise a coating layer coated on the amlodipine granule part.

In the inventive composition for preventing or treating cardiovascular disorders, the amlodipine granules and the mixture comprising losartan may respectively comprise pharmaceutically acceptable carriers or excipients. The pharmaceutically acceptable carriers or excipients may include microcrystalline cellulose, lactose, sodium citrate, calcium phosphate, glycine, starch, disintegrants (e.g., sodium starch glycolate, croscarmellose sodium and composite silicate) and granulating binders (e.g., polyvinylpyrrolidone, hydroxypropylmethylcellulose (HPMC), hydroxypropylcellulose (HPC), sucrose, gelatine and acacia gum). Further, the inventive composition may further comprise lubricants such as magnesium stearate, stearic acid, glyceryl behenate and talc.

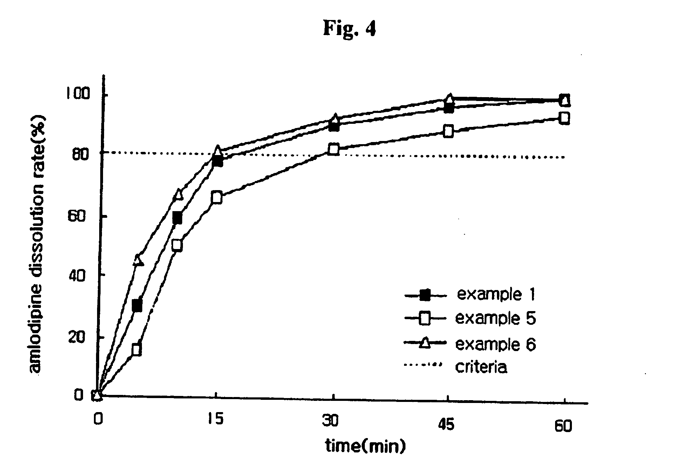

In a preferred embodiment of the present invention, the amlodipine granule part may comprise amlodipine or a pharmaceutically acceptable salt thereof and excipients in amounts corresponding to a weight ratio in the range of 1:10 to 1:60. When the weight ratio is less than 1:10, the dissolution rate of the composition becomes poor (*see* Example 5-7 and Fig. 4), and when more than 1:60, the composition becomes too bulky for easy administration.

Further, in the composition of the present invention, amlodipine or a pharmaceutically acceptable salt thereof and losartan or a pharmaceutically acceptable salt thereof may be used in amounts corresponding to a weight ratio in the range of 1:2.5 to 1:20, preferably 1:5 to 1:10.

The composition of the present invention may be administered in the form of a tablet, a capsule or multi-particles through various routes of oral administration including oral cavity, mouth and hypoglossus. However, it should be understood that the administration route of the inventive composition should be determined by the doctor in charge based on the patient's symptoms and requirements.

In accordance with a further aspect of the present invention, there is provided a method for preparing the inventive pharmaceutical composition comprising amlodipine or a pharmaceutically acceptable salt thereof and losartan or a pharmaceutically acceptable salt thereof, which comprises the steps of:
a) wet-granulating and drying a mixture of amlodipine or a pharmaceutically acceptable salt thereof and pharmaceutically acceptable excipients to obtain an amlodipine granule part; and
b) mixing the amlodipine granule part obtained in step a) with a mixture part comprising losartan or a pharmaceutically acceptable salt thereof and pharmaceutically acceptable excipients.

The following Examples are intended to further illustrate the present invention without limiting its scope.

### Example 1: Preparation of combined tablet I

- Amlodipine granule part -

| | |
|---|---|
| amlodipine camsylate | 7.84 mg (amlodipine 5 mg) |
| microcrystalline cellulose | 70.0 mg |
| calcium dihydrogenous phosphate | 60.0 mg |
| sodium starch glycolate | 12.0 mg |
| povidone | 3.0 mg |
| purified water | 70.0 mg |

- Losartan mixture part -

| | |
|---|---|
| losartan potassium | 50.0 mg |
| microcrystalline cellulose | 80.0 mg |
| sodium starch glycolate | 12.0 mg |
| magnesium stearate | 2.0 mg |

The ingredients of the amlodipine granule part were wet-granulated using 70.0 mg/tablet of purified water, passed through a 20 mesh, and dried to obtain the granule part having the specified amounts of the ingredient. The dried amlodipine granule part was mixed with the ingredients of the losartan mixture part according to the corresponding amounts, and the resulting mixture was formulated into a combined tablet having 5 mg of amlodipine and 50 mg of losartan.

### Example 2: Preparation of combined tablet II

- Amlodipine granule part -

| | |
|---|---|
| amlodipine camsylate | 7.84 mg (amlodipine 5 mg) |
| mannitol | 70.0 mg |
| calcium dihydrogenous phosphate | 60.0 mg |
| sodium starch glycolate | 12.0 mg |
| povidone | 3.0 mg |
| purified water | 70.0 mg |

- Losartan mixture part -

| | |
|---|---|
| losartan potassium | 50.0 mg |
| mannitol | 80.0 mg |
| sodium starch glycolate | 12.0 mg |
| magnesium stearate | 2.0 mg |

A combined tablet containing 5 mg of amlodipine and 50 mg of losartan was prepared by repeating the procedure of Example 1 except for using mannitol instead of microcrystalline cellulose.

### Example 3: Preparation of combined tablet III

- Amlodipine granule part -

| | |
|---|---|
| amlodipine camsylate | 15.68 mg |
| microcrystalline cellulose | 140.0 mg |
| calcium dihydrogenous phosphate | 120.0 mg |
| sodium starch glycolate | 24.0 mg |
| povidone | 6.0 mg |
| purified water | 140.0 mg |

- Losartan mixture part -

| | |
|---|---|
| losartan potassium | 50.0 mg |
| microcrystalline cellulose | 80.0 mg |
| sodium starch glycolate | 12.0 mg |
| magnesium stearate | 2.0 mg |

A combined tablet containing 10 mg of amlodipine and 50 mg of losartan was prepared by repeating the procedure of Example 1 except for using the ingredients described in amlodipine granule part twice the corresponding amounts, respectively.

### Example 4: Preparation of combined tablet IV

- Amlodipine granule part -

| | |
|---|---|
| amlodipine camsylate | 15.68 mg |
| microcrystalline cellulose | 140.0 mg |
| calcium dihydrogenous phosphate | 120.0 mg |
| sodium starch glycolate | 24.0 mg |
| povidone | 6.0 mg |
| purified water | 140.0 mg |

- Losartan mixture part -

| | |
|---|---|
| losartan potassium | 100.0 mg |
| microcrystalline cellulose | 160.0 mg |
| sodium starch glycolate | 24.0 mg |
| magnesium stearate | 4.0 mg |

A combined tablet containing 10 mg of amlodipine and 100 mg of losartan was prepared by repeating the procedure of Example 3 except for using the ingredients described in losartan mixture part twice the corresponding amounts, respectively.

### Example 5: Preparation of combined tablet V

- Amlodipine granule part -

| | |
|---|---|
| amlodipine camsylate | 7.84 mg (amlodipine 5 mg) |
| microcrystalline cellulose | 35.0 mg |
| calcium dihydrogenous phosphate | 30.0 mg |
| sodium starch glycolate | 6.0 mg |
| povidone | 1.5 mg |
| purified water | 35.0 mg |

- Losartan mixture part -

| | |
|---|---|
| losartan potassium | 50.0 mg |
| microcrystalline cellulose | 80.0 mg |
| sodium starch glycolate | 12.0 mg |
| magnesium stearate | 2.0 mg |

A combined tablet was prepared by repeating the procedure of Example I except for using the rest excipients in an amount of 10 parts by weight based on 1 part by weight of amlodipine camsylate in the procedure of preparing the amlodipine granule part.

### Example 6: Preparation of combined tablet VI

- Amlodipine granule part -

| | |
|---|---|
| amlodipine camsylate | 7.84 mg (amlodipine 5 mg) |
| microcrystalline cellulose | 231.0 mg |
| calcium dihydrogenous phosphate | 198.0 mg |
| sodium starch glycolate | 40.0 mg |
| povidone | 10.0 mg |
| purified water | 462.0 mg |

- Losartan mixture part -

| | |
|---|---|
| losartan potassium | 50.0 mg |
| microcrystalline cellulose | 80.0 mg |
| sodium starch glycolate | 12.0 mg |
| magnesium stearate | 2.0 mg |

A combined tablet was prepared by repeating the procedure of Example 1 except for using the rest excipients in an amount of 60 parts by weight based on 1 part by weight of amlodipine camsylate in the procedure of preparing the amlodipine granule part.

### Example 7: Preparation of combined tablet VII

- Amlodipine granule part -

| | |
|---|---|
| amlodipine camsylate | 15.68 mg (amlodipine 10 mg) |
| microcrystalline cellulose | 462.0 mg |
| calcium dihydrogenous phosphate | 396.0 mg |
| sodium starch glycolate | 80.0 mg |
| povidone | 20.0 mg |
| purified water | 924.0 mg |

- Losartan mixture part -

| | |
|---|---|
| losartan potassium | 50.0 mg |
| microcrystalline cellulose | 80.0 mg |
| sodium starch glycolate | 12.0 mg |
| magnesium stearate | 2.0 mg |

A combined tablet was prepared by repeating the procedure of Example 3 except for using the rest excipients in an amount of 60 parts by weight based on 1 part by weight of amlodipine camsylate in the procedure of preparing the amlodipine granule part.

### Reference example 8: Preparation of two-layer tablet

- Amlodipine tablet part -

| | |
|---|---|
| amlodipine camsylate | 7.84 mg (amlodipine 5 mg) |
| microcrystalline cellulose | 70.0 mg |
| calcium dihydrogenous phosphate | 60.0 mg |
| sodium starch glycolate | 12.0 mg |
| povidone | 3.0 mg |
| purified water | 70.0 mg |

- Losartan mixture part -

| | |
|---|---|
| losartan potassium | 50.0 mg |
| microcrystalline cellulose | 80.0 mg |
| sodium starch glycolate | 12.0 mg |
| magnesium stearate | 2.0 mg |

The ingredients described in amlodipine tablet part were wet-granulated using 70.0 mg/tablet of purified water, passed through a 20 mesh, and dried, according to the corresponding amounts. The dried amlodipine granule part was formulated into a tablet by using a two-layer tablet press machine (MRC-45, Sejong Pharmatech), the ingredients described in Losartan mixture part were added thereto according to the corresponding amounts, and the resulting mixture was formulated into a two-layer tablet containing 5 mg of amlodipine and 50 mg of losartan.

### Comparative Example 1: Preparation of direct-compression tablet of non-separated amlodipine and losartan

| | |
|---|---|
| amlodipine camsylate | 7.84 mg (amlodipine 5 mg) |
| microcrystalline cellulose | 150.0 mg |
| calcium dihydrogenous phosphate | 60.0 mg |
| sodium starch glycolate | 24.0 mg |
| povidone | 3.0 mg |
| losartan potassium | 50.0 mg |
| magnesium stearate | 2.0 mg |

All ingredients described in Example 1 were mixed together according to the corresponding amounts, and the mixture was formulated into a direct-compression tablet containing 5 mg of amlodipine and 50 mg of losartan.

### Test Example 1: Dissolution test of amlodipine

The combined tablet prepared in Example 1 and the tablet of a non-separated mixture prepared in Comparative Example 1 were each subjected to a drug dissolution test under the following conditions.

### - Test conditions -

Effluent: 500 ml of 0.01 N HCl (pH 2.0)
Dissolution-test system: USP paddle method, 75 rpm
Temperature: 37°C

### - Analytical conditions -

Column: stainless steel column (inner diameter: 4.6 mm, length: 25 cm) filled with octadecylsilanized silica gel for 5 µm liquid chromatography
Mobile phase: a mixture of methonol and 0.03M potassium dihydrogenous phosphate (600:400, v/v)
Detector: ultraviolet spectrophotometer (237 nm)
Flow rate: 1.5 ml/min
Injection volume: 20 µl

### - Criteria of Dissolution rate -

more than 80% at 30 mins.

### - Results -

As shown in Fig. 1, the combined tablet of amlodipine-losartan prepared in Example 1 exhibited an amlodipine dissolution rate two times higher as compared with that of the direct-compression tablet of a non-separated mixture prepared in Comparative Example 1. Further, the dissolution rate of the tablet prepared in Comparative Example 1 did not satisfy the required criteria, while that of the tablet of Example 1 met the criteria.

### Test Example 2: Dissolution test of losartan

The combined tablet prepared in Example I and the tablet of a non-separated mixture prepared in Comparative Example 1 were each subjected to a drug dissolution test under the following conditions.

### - Test conditions -

Effluent: purified water
Dissolution-test system: USP paddle method, 50 rpm
Temperature: 37°C

### - Analytical conditions -

Column: stainless steel column (inner diameter: 4.6 mm, length: 25 cm) filled with octadecylsilanized silica gel for 5 µm liquid chromatography
Mobile phase:
mobile phase A - phosphate buffer:acetonitrile (850:150, v/v)
mobile phase B - acetonitrile
concentration gradient system

| Time (min) | Mobile phase A % | Mobile phase B % |
|---|---|---|
| 0 | 80 | 20 |
| 10 | 40 | 60 |
| 11 | 80 | 20 |
| 15 | 80 | 20 |

Detector: ultraviolet spectrophotometer (250 nm)
Flow rate: 1.5 ml/min
Injection volume: 10 µl

### - Criteria of Dissolution rate -

more than 85% at 45 mins.

### - Results -

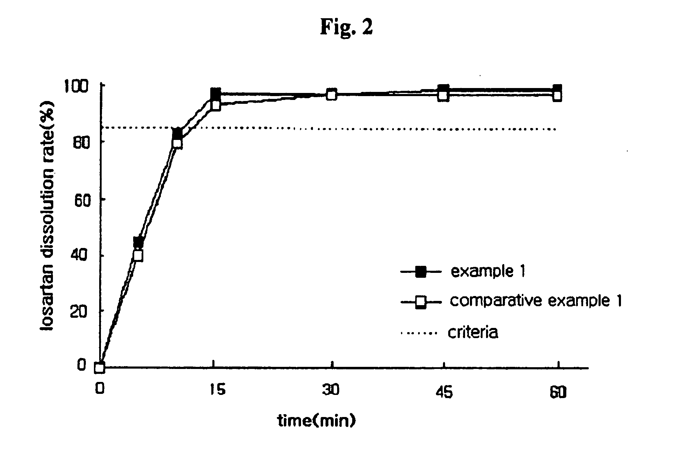

As shown in Fig. 2, the combined tablet of amlodipine-losartan prepared in Example 1 and the tablet of a non-separated mixture prepared in Comparative Example 1 did not show any significant difference in the dissolution rates of losartan, and all the two tablets met the criteria. This suggests that the separate granulation of amlodipine does not affect the dissolution rate of losartan.

### Test Example 3: Dissolution test of amlodipine for the formulations of Examples 1 to 4

The combined tablets of Examples 1 to 4, which were prepared by using a separated granule, respectively, were each subjected to drug dissolution test under the same test and analytical conditions of Test Example 1.

### - Results -

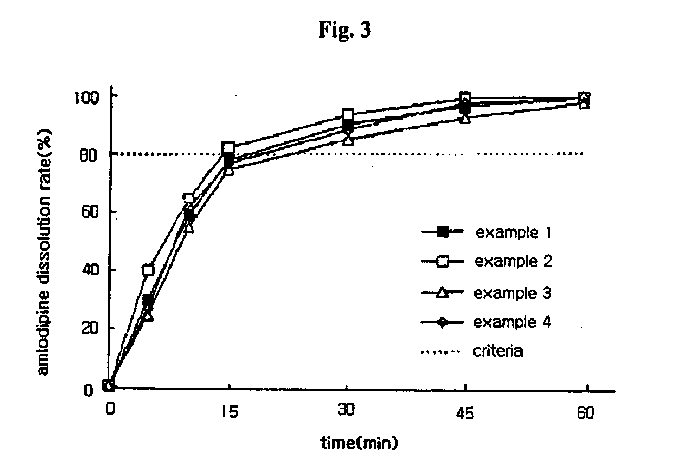

As can be seen in Fig. 3, the combined tablets prepared in Examples exhibited similar amlodipine dissolution rates regardless of the difference in the content or kind of ingredients.

### Test Example 4: Dissolution test of amlodipine for the formulations of Examples 1, 5 and 6

The combined tablets prepared in Examples 1, 5 and 6 were each subjected to drug dissolution test under the same test and analytical conditions of Test Example 1.

### - Results -

As shown in Fig. 4, the tablet obtained in Example 6, which was prepared by using the rest excipients in an amount of 60 parts by weight based on 1 part by weight of amlodipine camsylate, exhibited a similar amlidipine dissolution rate to that of the formulation obtained in Example 1, while the tablet of Example 5, which was prepared by using the rest excipients in an amount of 10 parts by weight based on 1 part by weight of amlodipine camsylate, showed a slightly lower dissolution rate than that of the formulation of Example 1. The dissolution rate at 30 min of the formulation obtained in Example 5 is 80%, which met the criteria, and accordingly, it can be expected that the dissolution rate of the inventive formulation would become unsatisfactory when the amount of the rest excipients is less than 10 parts by weight.

### Test Example 5: Dissolution test of amlodipine for the formulations of Examples 1 and Reference Example 8

The combined tablet obtained in Example 1 and the two-layer tablet obtained in Reference Example 8 were each subjected to drug dissolution test under the same test and analytical conditions of Test Example 1.

### - Results -

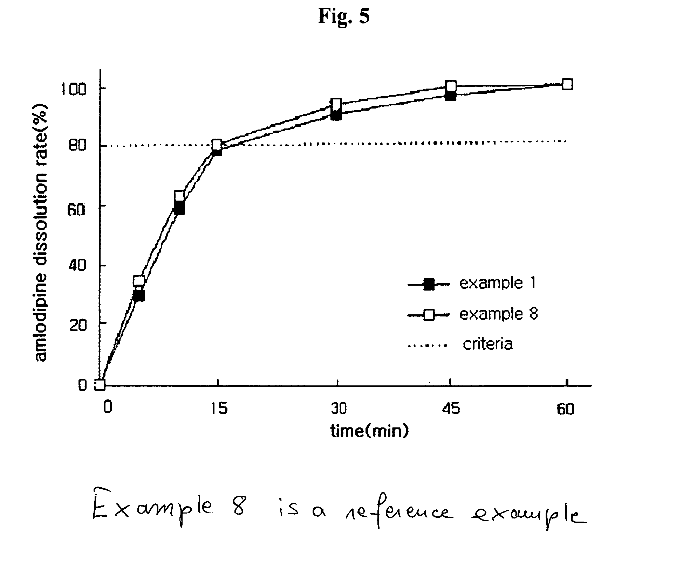

As shown in Fig 5, the combined tablet obtained in Example 1 and the two-layer tablet obtained in Reference Example 8 exhibited similar dissolution rates regardless of the difference in the forms of the formulation. Accordingly, this suggest that the inventive formulation prepared by using a separated granule of amlodipine or a pharmaceutically acceptable salt thereof would exhibit a satisfactory dissolution rate irrespective of the formulation form.

### Test Example 6: Stability test of amlodipine

Stability test was performed for the combined tablet obtained in Example 1, which was prepared by using a separated granule, and the tablet of a non-separated mixture obtained in Comparative Example 1 under the following conditions.
Incubation conditions: HDPE bottle at 40°C/75% relative humidity
Incubation time: 0, 1, 2, 4 and 6 months
Subject of test: amlodipine
Analytical conditions: the analytical conditions of Example 1

The results are shown in Table 1.

**Table 1**

| Formulation | 0 | 1 month | 2 months | 4 months | 6 months |
|---|---|---|---|---|---|
| Example 1 | 100.3% | 100.2% | 99.6% | 98.9% | 99.1% |
| Comparative Example 1 | 100.2% | 97.8% | 94.9% | 90.3% | 85.7% |

As shown in Table 1, the combined tablet obtained in Example 1, which was prepared by using a separated granule, exhibited a high drug stability as compared with the tablet of a non-separated mixture obtained in Comparative Example 1.

As described above, the inventive composition comprising amlodipine or a pharmaceutically acceptable salt thereof and losartan or a pharmaceutically acceptable salt thereof can achieve improved therapeutic effects for cardiovascular disorders. Further, the inventive composition prepared by using a separated granule of amlodipine or a pharmaceutically acceptable salt thereof provides improved dissolution rate and stability relative to the composition of a non-separated mixture of amlodipine and losartan when administered.

## Claims

1. A composition for use in preventing or treating cardiovascular disorders comprising amlodipine or a pharmaceutically acceptable salt thereof, losartan or a pharmaceutically acceptable salt thereof, and pharmaceutically acceptable excipients, wherein amlodipine or a pharmaceutically acceptable salt thereof and losartan or a pharmaceutically acceptable salt thereof are physically separated from each other, said composition being obtainable by a method comprising the steps of granulating amlodipine or a pharmaceutically acceptable salt thereof to obtain an amlodipine granule part, and mixing the amlodipine granule part with a mixture part comprising losartan or a pharmaceutically acceptable salt thereof.

2. The composition according to claim 1 for use in preventing or treating cardiovascular disorders according to claim 1, which further comprises a coating layer formed on the amlodipine granule part.

3. The composition according to claim 1 for use in preventing or treating cardiovascular disorders according to claim 1, wherein the amlodipine granule part and the mixture part further comprise pharmaceutically acceptable excipients.

4. The composition according to claim 3 for use in preventing or treating cardiovascular disorders according to claim 1, wherein the amlodipine granule part comprises amlodipine or a pharmaceutically acceptable salt thereof and pharmaceutically acceptable excipients in amounts corresponding to a weight ratio in the range of 1:10 to 1:60.

5. The composition according to claim 1 for use in preventing or treating cardiovascular disorders according to claim 1, wherein the pharmaceutically acceptable salt of amlodipine is amlodipine camsylate.

6. The composition according to claim 1 for use in preventing or treating cardiovascular disorders according to claim 1, wherein the pharmaceutically acceptable salt of losartan is losartan potassium.

7. The composition according to claim 1 for use in preventing or treating cardiovascular disorders according to claim 1, wherein the cardiovascular disorders are selected from the group consisting of angina pectoris, hypertension, artery vasospasm, deep vein thrombosis, cardiac hypertrophy, cerebral infarct, congestive heart failure and myocardial infarction.

8. A method for preparing the composition of claim 1, which comprises the steps of:
a) wet-granulating and drying a mixture of amlodipine or a pharmaceutically acceptable salt thereof and pharmaceutically acceptable excipients to obtain an amlodipine granule part; and
b) mixing the amlodipine granule part obtained in step a) with a mixture part comprising losartan or a pharmaceutically acceptable salt thereof and pharmaceutically acceptable excipients.

## Patentansprüche

1. Zusammensetzung für die Verwendung im Vorbeugen oder Behandeln von Herz-Kreislauf-Erkrankungen, umfassend Amlodipin oder ein pharmazeutisch verträgliches Salz davon, Losartan oder ein pharmazeutisch verträgliches Salz davon, und pharmazeutisch verträgliche Hilfsstoffe, wobei Amlodipin oder ein pharmazeutisch verträgliches Salz davon und Losartan oder ein pharmazeutisch verträgliches Salz davon physisch voneinander getrennt sind, wobei besagte Zusammensetzung durch ein Verfahren erhältlich ist, umfassend die Schritte Granulieren von Amlodipin oder einem pharmazeutisch verträglichen Salz davon, um einen Amlodipin-Granulat-Teil zu erhalten, und Mischen des Amlodipin-Granulat-Teils mit einem Mischungsteil, der Losartan oder ein pharmazeutisch verträgliches Salz davon umfasst.

2. Zusammensetzung nach Anspruch 1 für die Verwendung im Vorbeugen oder Behandeln von Herz-Kreislauf-Erkrankungen nach Anspruch 1, die zusätzlich eine auf dem Amlodipin-Granulat-Teil gebildete Oberflächenbeschichtung umfasst.

3. Zusammensetzung nach Anspruch 1 für die Verwendung im Vorbeugen oder Behandeln von Herz-Kreislauf-Erkrankungen nach Anspruch 1, wobei der Amlodipin-Granulat-Teil und der Mischungsteil zusätzlich pharmazeutisch verträgliche Hilfsstoffe umfassen.

4. Zusammensetzung nach Anspruch 3 für die Verwendung im Vorbeugen oder Behandeln von Herz-Kreislauf-Erkrankungen nach Anspruch 1, wobei der Amlodipin-Granulat-Teil Amlodipin oder ein pharmazeutisch verträgliches Salz davon und pharmazeutisch verträgliche Hilfsstoffe umfasst, in Mengen, die einem Gewichtsverhältnis im Bereich von 1:10 bis 1:60 entsprechen.

5. Zusammensetzung nach Anspruch 1 für die Verwendung im Vorbeugen oder Behandeln von Herz-Kreislauf-Erkrankungen nach Anspruch 1, wobei das pharmazeutisch verträgliche Salz von Amlodipin Amlodipincamsylat ist.

6. Zusammensetzung nach Anspruch 1 für die Verwendung im Vorbeugen oder Behandeln von Herz-Kreislauf-Erkrankungen nach Anspruch 1, wobei das pharmazeutisch verträgliche Salz von Losartan Kaliumlosartan ist.

7. Zusammensetzung nach Anspruch 1 für die Verwendung im Vorbeugen oder Behandeln von Herz-Kreislauf-Erkrankungen nach Anspruch 1, wobei die Herz-Kreislauf-Erkrankungen gewählt werden aus der Gruppe, die besteht aus Angina pectoris, Hypertension, arterieller Vasospasmus, tiefe Venenthrombose, kardiale Hypertrophie, Hirninfarkt, kongestives Herzversagen und Myokardininfarkt.

8. Verfahren zum Herstellen der Zusammensetzung nach Anspruch 1, welches die Schritte umfasst:
a) Nassgranulieren und Trocknen eines Gemischs aus Amlodipin oder einem pharmazeutisch verträglichen Salz davon und pharmazeutisch verträglichen Hilfsstoffen, um einen Amlodipin-Granulat-Teil zu erhalten; und
b) Mischen des in Schritt a) erhaltenen Amlodipin-Granulat-Teils mit einem Mischungsteil, der Losartan oder ein pharmazeutisch verträgliches Salz davon und pharmazeutisch verträgliche Hilfsstoffe umfasst.

## Revendications

1. Composition destinée à être utilisée dans la prévention ou le traitement de maladies cardiovasculaires, comprenant de l'amlodipine ou un sel pharmaceutiquement acceptable de celle-ci, du losartan ou un sel pharmaceutiquement acceptable de celui-ci, et des excipients pharmaceutiquement acceptables, dans laquelle l'amlodipine ou un sel pharmaceutiquement acceptable de celle-ci et le losartan ou un sel pharmaceutiquement acceptable de celui-ci sont physiquement séparés l'un de l'autre, ladite composition pouvant être obtenue grâce à une procédé comprenant les étapes de granulation de l'amlodipine ou d'un sel pharmaceutiquement acceptable de celle-ci pour obtenir une fraction de granules d'amlodipine, et de mélange de la fraction de granules d'amlodipine avec une fraction de mélange comprenant du losartan ou un sel pharmaceutiquement acceptable de celui-ci.

2. Composition selon la revendication 1 destinée à être utilisée dans la prévention ou le traitement de maladies cardiovasculaires selon la revendication 1, comprenant en outre une couche de revêtement formée sur la fraction de granules d'amlodipine.

3. Composition selon la revendication 1 destinée à être utilisée dans la prévention ou le traitement de maladies cardiovasculaires selon la revendication 1, dans laquelle la fraction de granules d'amlodipine et la fraction de mélange comprennent en outre des excipients pharmaceutiquement acceptables.

4. Composition selon la revendication 3 destinée à être utilisée dans la prévention ou le traitement de maladies cardiovasculaires selon la revendication 1, dans laquelle la fraction de granules d'amlodipine comprend de l'amlodipine ou un sel pharmaceutiquement acceptable de celle-ci et des excipients pharmaceutiquement acceptables dans des quantités correspondant à un ratio massique compris entre 1:10 et 1:60.

5. Composition selon la revendication 1 destinée à être utilisée dans la prévention ou le traitement de maladies cardiovasculaires selon la revendication 1, dans laquelle le sel d'amlodipine pharmaceutiquement acceptable est un camsilate d'amlodipine.

6. Composition selon la revendication 1 destinée à être utilisée dans la prévention ou le traitement de maladies cardiovasculaires selon la revendication 1, dans laquelle le sel de losartan pharmaceutiquement acceptable est du losartan potassique.

7. Composition selon la revendication 1 destinée à être utilisée dans la prévention ou le traitement de maladies cardiovasculaires selon la revendication 1, dans laquelle les maladies cardiovasculaires sont sélectionnées parmi le groupe constitué par l'angine de poitrine, l'hypertension, le vasospasme artériel, la thrombose veineuse profonde, la cardiomyopathie hypertrophique, l'infarctus cérébral, l'insuffisance cardiaque congestive et l'infarctus du myocarde.

8. Procédé de préparation de la composition selon la revendication 1, comprenant les étapes suivantes :
a) granulation par voie humide et séchage d'un mélange d'amlodipine ou d'un sel pharmaceutiquement acceptable de celle-ci et d'excipients pharmaceutiquement acceptables pour obtenir une fraction de granules d'amlodipine ; et
b) mélange de la fraction de granules d'amlodipine obtenue à l'étape a) avec une fraction de mélange comprenant du losartan ou un sel pharmaceutiquement acceptable de celui-ci et des excipients pharmaceutiquement acceptables.
